# EUROPEAN PATENT SPECIFICATION

(11) **EP 3 621 529 B1**
(45) Date of publication and mention of the grant of the patent: **28.10.2026**
(21) Application number: 18730501.6
(22) Date of filing: 11.05.2018
(51) Int. Cl.: A61B 17/00, A61B 17/08, A61F 2/24

(54) **LONG ARM VALVE REPAIR CLIP**
KLAPPENREPARATURCLIP MIT LANGEN ARMEN
PINCE DE RÉPARATION DE VALVULE À BRAS LONG

(30) Priority: 12.05.2017 US 201762505810 P
(43) Date of publication of application: 18.03.2020
(73) Proprietor: Evalve, Inc., Santa Clara, CA 95054 (US)
(72) Inventor: ABUNASSAR, Chad, San Francisco, CA 94103 (US); GARCIA, Jessie, Newark, CA 94560 (US); MAHMOUD, Tamer, Sunnyvale, CA 94087 (US); CHU, Brandon, San Francisco, CA 94122 (US); PRABHU, Santosh, Sunnyvale, CA 94086 (US)
(74) Representative: J A Kemp LLP
(86) International application number: PCT/US2018/032446
(87) International publication number: WO 2018/209313

(56) References cited:
- US-A1- 2017 020 521

## Description

### Background

### Cross-Reference to Related Applications

This application claims the benefit of U.S. Provisional Patent Application No. 62/505,810, filed May 12, 2017.

### Field of Disclosed Subject Matter

The disclosed subject matter is directed to medical devices for the endovascular, percutaneous or minimally invasive surgical treatment of bodily tissues, such as tissue approximation or valve repair. More particularly, the present invention relates to repair of valves of the heart and venous valves.

Surgical repair of bodily tissues can involve tissue approximation and fastening of such tissues in the approximated arrangement. When repairing valves, tissue approximation includes coapting the leaflets of the valves in a therapeutic arrangement which can then be maintained by fastening or fixing the leaflets. Such coaptation can be used to treat regurgitation, which commonly occurs in the mitral valve and in the tricuspid valve.

Mitral valve regurgitation is characterized by retrograde flow from the left ventricle of a heart through an incompetent mitral valve into the left atrium. During a normal cycle of heart contraction (systole), the mitral valve acts as a check valve to prevent flow of oxygenated blood back into the left atrium. In this way, the oxygenated blood is pumped into the aorta through the aortic valve. Regurgitation of the mitral valve can significantly decrease the pumping efficiency of the heart, placing the patient at risk of severe, progressive heart failure.

Mitral valve regurgitation can result from a number of different mechanical defects in the mitral valve or the left ventricular wall. The valve leaflets, the valve chordae which connect the leaflets to the papillary muscles, the papillary muscles or the left ventricular wall can be damaged or otherwise dysfunctional. Commonly, the valve annulus can be damaged, dilated, or weakened limiting the ability of the mitral valve to close adequately against the high pressures of the left ventricle.

### Description of Related Art

Treatments for mitral valve regurgitation rely on valve replacement or repair including leaflet and annulus remodeling, the latter generally referred to as valve annuloplasty. Another technique for mitral valve repair, which relies on suturing adjacent segments of the opposed valve leaflets together is referred to as the "bow-tie" or "edge-to-edge" technique. Devices, and systems should preferably not require open chest access and be capable of being performed either endovascularly, i.e., using devices, such as a catheter, which are advanced to the heart from a point in the patient's vasculature remote from the heart. Preferably, such devices and systems allow for repositioning and optional removal of a fixation device (i.e., valve repair clip) prior to fixation to ensure optimal placement. Such devices and systems likewise can be useful for repair of tissues in the body other than heart valves.
US 2017/020521 discloses a tissue gripping device formed from a shape-memory material, and having a base section, a first arm, and a second arm disposed opposite the first arm, each arm having a first end coupled to the base section and a free end extending from the base section. The arms of the tissue gripping device are configured to resiliently flex toward a relaxed configuration in a distal direction as the tissue gripping device is moved from a pre-deployed configuration toward a deployed configuration. The tissue gripping device is usable in a method for gripping tissue. The method includes positioning the tissue gripping device near target tissue and moving the tissue gripping device from a pre-deployed configuration toward a deployed configuration in order to grip the target tissue.

### Summary

The purpose and advantages of the disclosed subject matter will be set forth in and apparent from the description that follows, as well as will be learned by practice of the disclosed subject matter. Additional advantages of the disclosed subject matter will be realized and attained by the methods and systems particularly pointed out in the written description and claims hereof, as well as from the appended drawings.

To achieve these and other advantages and in accordance with the purpose of the disclosed subject matter, as embodied and broadly described, the disclosed subject matter is directed to a fixation device for treating a patient.

In accordance with the disclosed subject matter, there is provided a fixation device as defined in the claims.

The length L₁ can be about 8.9mm (0.35 inches). The central portion can have a generally U-shaped configuration with the distal end disposed between the respective central portion-gripping element interfaces. Each fixation element can be rotatable about a respective axis point, and each fixation element can have an elongate portion defining a respective reference axis. A length "L₂" can be defined along the respective reference axis between the respective axis point and the respective second end of each fixation element. The length L₁ can be at least the length of L₁. The ratio of the lengths L₂:L₁ can be about 1.35:1. Each gripping element can have a plurality of rows of fixation elements. For example, each gripping element can nave at least four rows of friction elements extending from a gripping element surface. Each row can include one or more friction elements.

### Brief Description of the Figures

FIG. 1 is a perspective view of an exemplary embodiment a fixation device in accordance with the disclosed subject matter.
FIG. 2 is a front view of a pair of gripping elements and a central portion therebetween.
FIG. 3 is a front view of a portion of the fixation device of FIG. 1.
FIGS. 4A-4D are perspective views of a various embodiments of the gripping elements.
FIG. 5 is a front view of a modified embodiment of the fixation device of FIG. 1.
FIG. 6 is a front view of an embodiment of the fixation device of FIG. 1 on the distal end of a catheter.
FIG. 7 is a comparative front view of a portion of the fixation device of FIG. 1.
FIG. 8 is a comparative front view of a portion of the fixation device of FIG. 1.
FIGS. 9A-9C are front views of a portion of the fixation device of FIG. 1 at various positions.
FIG. 10 is a side view of a fixation element of the fixation device of FIG. 1.
FIGS. 11A-11B are comparative side views of a fixation element of the fixation device of FIG. 1.
FIG. 12A is a perspective view of a L-Lock component in combination with the fixation device of FIG. 1.
FIG. 12B is a front view of the L-Lock component.
FIG. 12C is a front view of an embodiment of the fixation device of FIG. 1 on the distal end of a catheter.
FIG. 13 is a front view of an embodiment of the fixation device of FIG. 1.
FIG. 14 is a perspective view of an embodiment of the gripping element of FIG. 2.

### Detailed Description

Reference will now be made in detail to the various exemplary embodiments of the disclosed subject matter, exemplary embodiments of which are illustrated in the accompanying drawings,

The device of the disclosed subject matter provides an edge-to-edge transcatheter valve repair option for patients having various conditions, including regurgitant mitral valves or tricuspid valves. In grasping tissue and leaflet capture for mitral valve disease, there are occasions where a particularly challenging anatomy can be associated with larger dynamic gaps between leaflet tips that cannot be effectively captured by previous designs. In order to improve ease of achieving procedural success in these cases, a fixation device (i.e., valve repair clip) having modified fixation elements and gripping elements is disclosed herein. The fixation device disclosed herein is able to bridge larger gaps in functional mitral regurgitation (FMR) while also providing more reliable leaflet capture in cases of dynamic, chaotic, or overly severe degenerative mitral regurgitation (DMR), such as in cases of Barlow's Syndrome.

The device of the disclosed subject matter herein can provide for an edge-to-edge valve repair option for degenerative mitral valve regurgitation (DMR) and functional mitral valve regurgitation (FMR) cases. In addition, the device of the disclosed subject matter can be used in tricuspid valve treatments.

To address various problems associated with treating severely diseased tricuspid valve anatomies, a fixation device is disclosed herein with improved configurations for enhanced performance.

Generally, and as set forth in greater detail below, the disclosed subject matter provided herein includes a fixation device for engaging tissue including a pair of fixation elements, each fixation element has a first end and a second end opposite the first end, the first ends are moveable between a closed position and an open position. The fixation device further includes a pair of gripping elements. Each gripping element is moveable with respect to a respective fixation element and disposed in opposition to at least a portion of the respective fixation element to capture tissue therebetween. The fixation device further includes a central portion operatively connected to each gripping element at a respective central portion-gripping element interface. The central portion has a distal end and a width "W₁" proximate the distal end. Each gripping element has a free end opposite its respective central portion-gripping element interface and a length "L₁" defined between the respective central portion-gripping element interface and the free end. The length L₁ of the fixation elements is at least about three times the width W₁. Additionally, or alternatively, and in accordance with another aspect of the disclosed subject matter the distal end of the central portion can define a reference plane perpendicular to a central axis of the central portion wherein, a height "H₁" can be defined by a vertical dimension between the reference plane and the central portion-gripping element interfaces, and the length L₁ can be at least 3.8 times the height H₁.

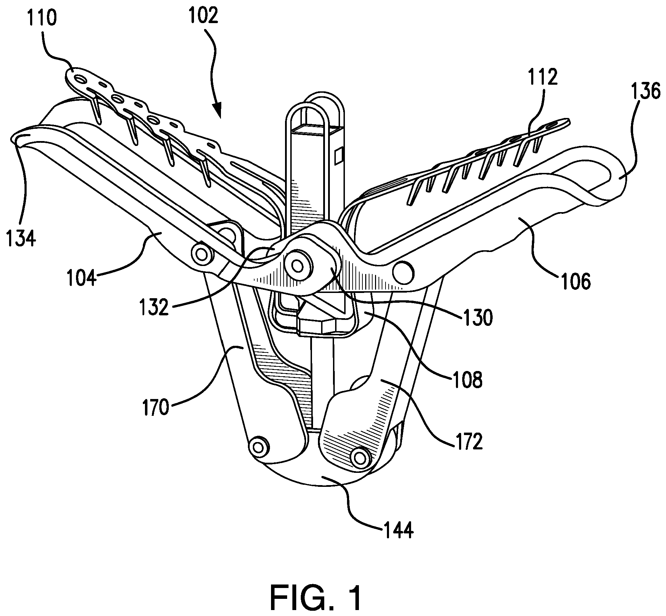

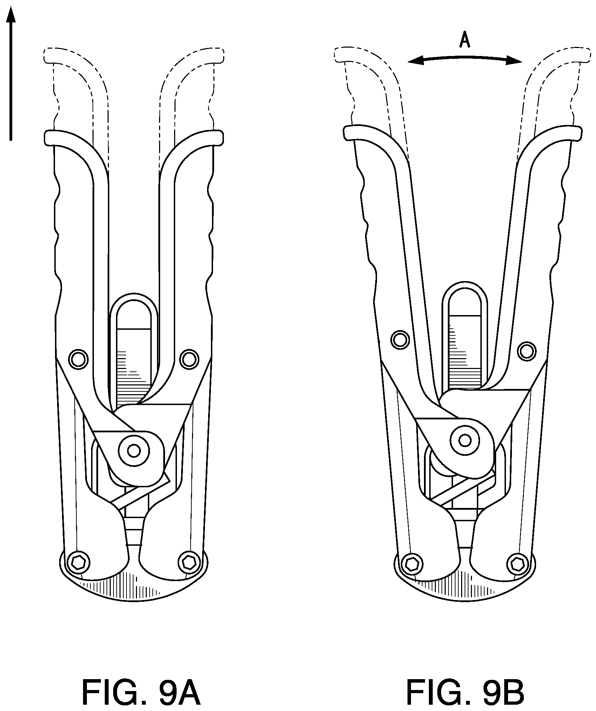

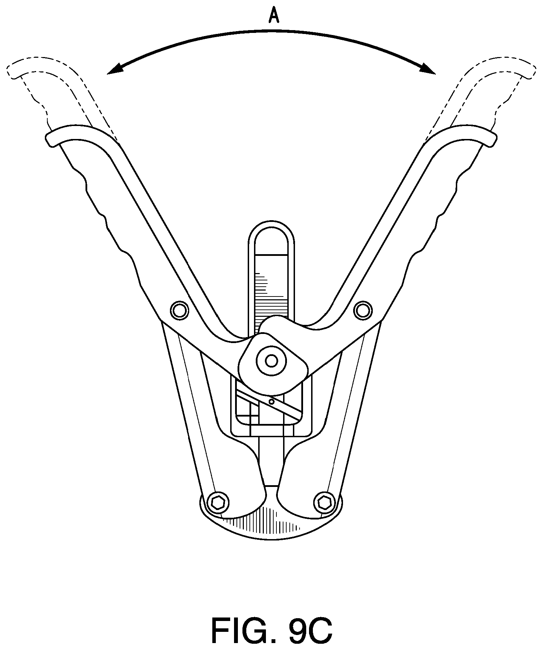

Referring to FIGS. 1-3 for the purpose of illustration and not limitation, a fixation device 102 for engaging tissue is disclosed herein and includes a pair of fixation elements 104, 106 each having a first end 130,132 and a second end 134, 136 opposite the first end, the first ends 330, 132 being movably coupled together such that the fixation elements are moveable between a closed position and an open position. For the purpose of illustration and not limitation an exemplary embodiment of a closed position is shown in FIG. 9A and an exemplary embodiment of an open position is shown in FIG. 9C.

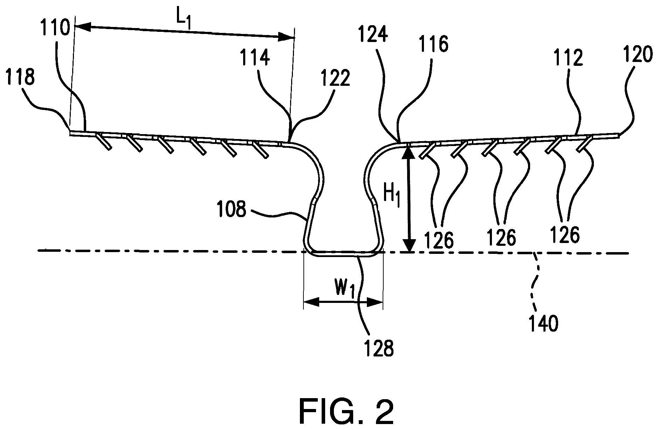

As embodied herein, and in accordance with the disclosed subject matter, the fixation device further includes a pair of gripping elements 110, 112, each gripping element moveable with a respective fixation element 104, 106, and disposed in opposition to at least a portion of the respective fixation element to capture tissue therebetween. The fixation device further includes a central portion 108 operatively connected to each of the gripping elements 110, 112 at respective central portion-gripping element interface 122, 124. With reference to FIG. 2, the central portion 108 has a distal end 128 and a width "W₁" proximate the distal end. Each gripping element has a free end 118, 120 opposite its respective central portion-gripping element interface 122, 124, wherein a length "L₁" is defined between the respective central portion-gripping element interface 122, 124 and the free end 118, 120. As disclosed herein, the length L₁ is at least about three times the width W₁ . The ratio of the width to the length W₁:L₁ is about 1:3.

In accordance with another aspect of the disclosed subject matter, the distal end can define a reference plane 140 perpendicular to a central axis of the central portion. A height "H₁" can be defined by a vertical dimension between the reference plane 3 40 and the central portion-gripping element interfaces 122, 124. The ratio of the height to the length H₁:L₁ is about 1:1.8. The length L₁ can be about 8.9mm (0.35 inches).

The central portion 108 can be a than each of the gripper elements or can be formed integrally as a single piece with the gripper elements as embodied herein. For example, the central portion can have a generally U-shaped configuration with the distal end 128 disposed between the central portion-gripping element interfaces 122,124. As embodied herein for the purpose of illustration and not limitation, the central portion 108 can be connected to the gripping elements. However, the central portion is not limited to a U-shaped configuration and can be a component having any number of shapes, sizes, and functions.

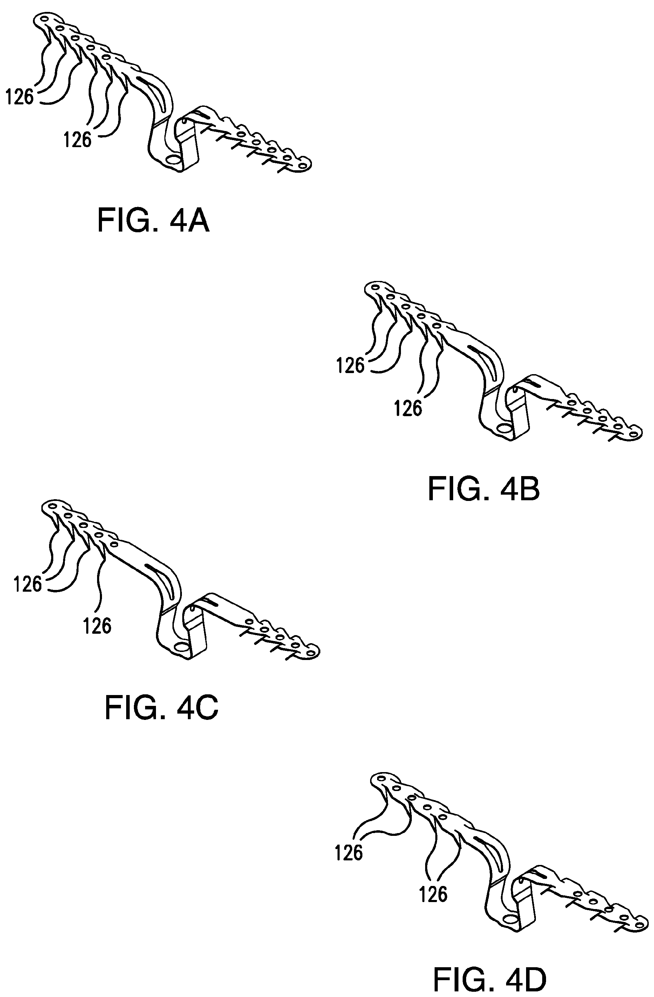

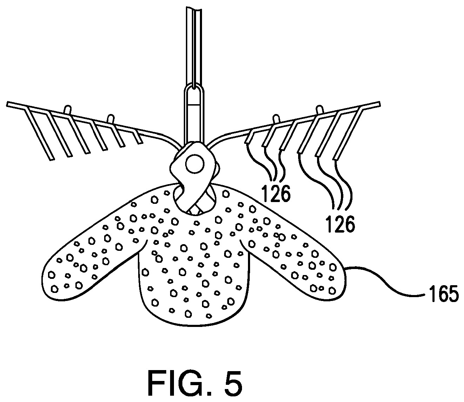

Further in accordance with the disclosed subject matter each gripper element can include one or more friction elements. As embodied herein, each gripping element includes a plurality of friction elements, such as in rows. Referring now to FIGS. 4 and 5 for the purpose of illustration and not limitation, various exemplary embodiments of the friction elements 126 are provided. As depicted, a plurality of friction elements 126 can be provided. For example, each gripping element 110, 112 can have at least four rows of friction elements 126 extending from a gripping element surface. Each row can include one or more friction elements 126. For the purpose of illustration and not limitation, FIG. 4A shows six rows of friction elements 126 and FIG. 4B shows five rows of friction elements. Six rows of friction elements 126 on either side of the gripping element (12 total), can allow for improved tissue engagement during leaflet capture. This gripping element design increases the assurance that single device leaflet attachment (SLDA) will not occur during a procedure, which constitutes an adverse safety risk. If the fixation device requires adjustment after an initial leaflet capture, the fixation elements can be opened, the gripping element can be raised vertically, and tissue can disengage from the fixation device, facilitating re-grasp and capture.

Alternatively, each gripping element can have fewer rows of friction elements as appropriate, such as 1 row, 2 rows, 3 row, or 4 rows, as shown in FIG. 4C and 4D. Fewer rows can improve the ability of the device to release tissue during adjustment after an initial leaflet capture. In comparing FIGS. 4A-4C, in decreasing order, tissue engagement is expected to be highest in FIG. 4A, then FIG. 4B, then FIG. 4C, and in decreasing order, ease of leaflet release is expected to be highest in FIG. 4C, the FIG. 4B, then FIG, 4A.

Friction elements can be distributed near the free ends of the gripping elements 118, 120, as shown in FIGS. 4B and 5B, or the rows can be redistributed along the length of the gripping element, as shown in FIG. 4D. The redistributed rows can provide an alternate performance compromise between leaflet engagement and leaflet release. Spacing of gripping elements may be biased (non-uniform) to provide performance improvements in either leaflet capture, leaflet release, or both.

The length of each friction element 126 can be constant, as shown in FIGS. 1, 2 and 4. Alternatively, the friction elements 126 can be provided with varying sizes. For example, the length can increase in length toward the free ends of the gripping elements 118, 120, as shown in FIG. 5. The increase in length can be linear or parabolic. The variation in length as shown for FIG. 5C is for purpose of illustration not limitation, and the variation in length can be significantly smaller than shown. This length variation may be continuous or staggered in alternating rows of gripping elements to produce an undulating profile.

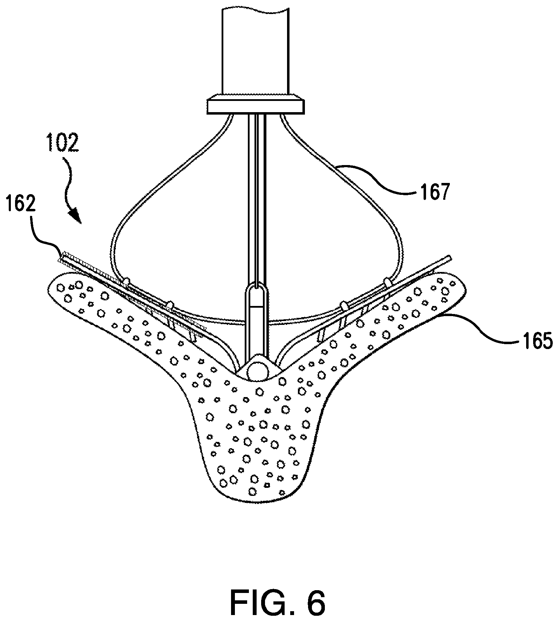

Referring now to FIG. 6, the fixation device can have a cover over the gripping elements. The cover can be made of a variety of suitable materials. For purpose of comparison only, a cover 162 is shown on only one gripping element in FIG. 6 whereas the other gripping element is depicted without a cover. However, each gripping element can have a cover 162, The cover can be configured for friction elements 126 to protrude at varying degrees along the length of the gripping elements 110, 112. For example, the friction elements can have a linearly or parabolically increasing protruded length when moving toward the free ends by varying the gripping element cover dimensions. The cover 162 can cover the gripping element friction elements 126 such that only a portion of the friction elements 126 protrudes through the cover. Increased friction element height protruding through the cover can enhanced tissue fixation. The height can be varied along the length of the gripping element flat section such that tissue fixation is maximized proximate the free end of the gripping dement minimized proximate the first end of the gripper. For example, the cover could be a type of film with a varied thickness along the length of the gipping element. The cover 162 can form a rectangular sheath around the gripper elements so only part of the friction element length protrudes through the cover thickness. This configuration can increase the combination of reliable leaflet capture and the leaflet release. As further depicted in FIG. 6, a cover 165 can also be provided over the fixation elements 104, 106.
The cover 165 can be made of polyester fabric.

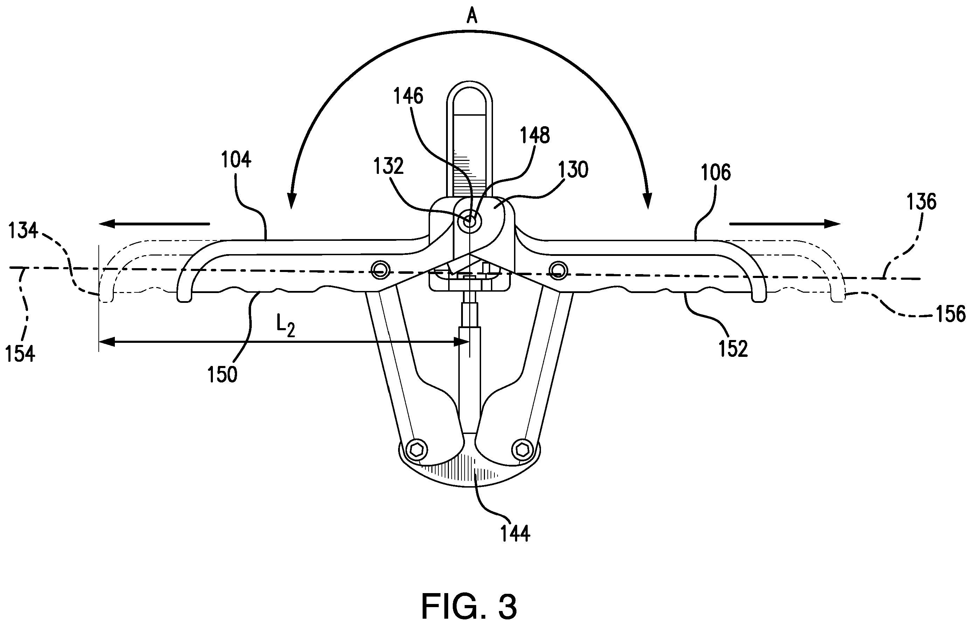

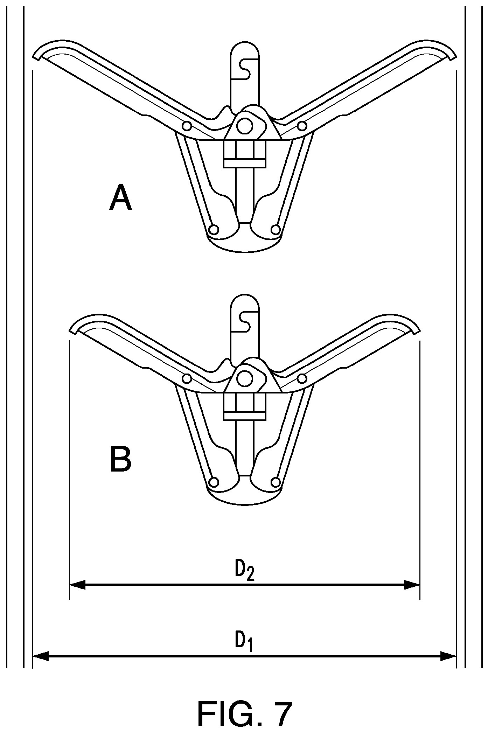

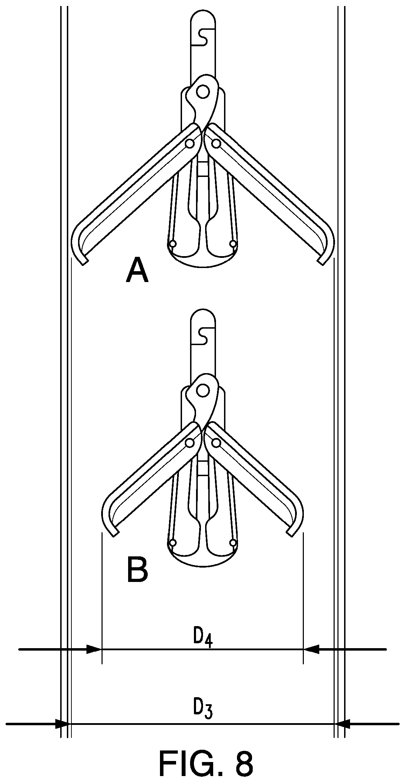

Further in accordance with the disclosed subject matter, the fixation elements also can be provided with increased length as compared to conventional fixation devices. The fixation device can further include a base 144 operatively connected to the fixation elements. Each fixation element 134, 136 can be rotatable about a respective axis point 146, 148. Each fixation element 134, 136 can have an elongate portion 150, 152 defining a respective reference axis 134,136. A length "L₂" can be defined along the respective reference axis 154, 156 between the respective axis point 146, 148 and the respective second end 134, 136 of each fixation element. For purpose of discussion and comparison with a conventional device having a shorter length fixation element, the increased length of each fixation element of the disclosed subject matter is depicted in dashed lines in FIG. 3 as well as FIGS. 9A-9C. The length L₂ of the fixation element can be at least about the length of L₁. For example, the ratio of the lengths L₂:L₁ can be about 1.35:1. For purpose of comparison, FIGS. 7 and 8 depict the fixation elements of the disclosed subject matter labeled as embodiment "A" whereas a conventional fixation device is depicted as embodiment "B".

In FIG. 7, the fixation devices are in an open position, wherein tissue capture can occur in this position. A dimension can be defined as the horizontal distance between the second ends of the fixation elements 134, 136. For embodiment A, dimension D₁ can be about 24 millimeters, whereas for embodiment B, dimension D2 can be about 19 millimeters. In FIG. 8, the fixation elements are in an inverted position, wherein tissue release and device retraction can occur in this position. For embodiment A, dimension D3 can be about 15 millimeters, whereas for embodiment B, dimension D4 can be about 11 millimeters.

Referring now to FIGS. 9A-C, various positions of the fixation device 102 are depicted for purpose of illustration and not limitation. Elongated fixation elements of the disclosed subject matter are illustrated in dashed lines for comparison to shorter fixation elements. In FIG. 9A the fixation device is in the closed position, wherein the fixation elements are positioned vertically. FIGS. 9B and 9C illustrate the fixation elements positioned with an angle A between the two elements. In FIG. 9B, A is 10 degrees and in FIG. 9C A is 60 degrees.

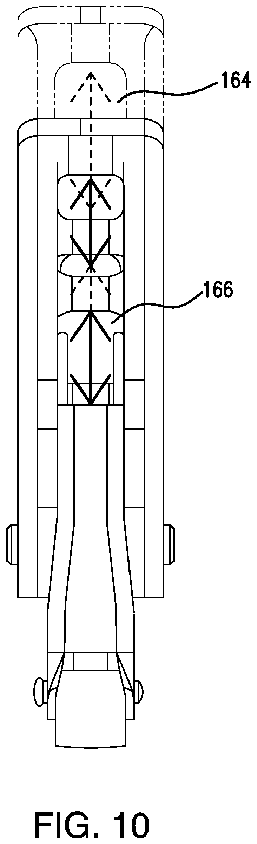

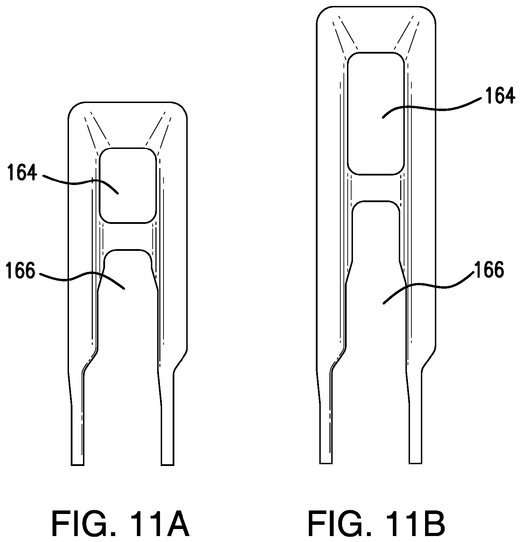

Referring now to FIGS. 10 and 11, side views of the fixation elements are shown wherein a first opening 164 and a second opening 166 have an increased length in an elongated fixation element of the disclosed subject matter, as compared to a shorter fixation element. In FIG. 10, arrows illustrate increased length of the openings 164, 166, while their respective widths remain constant. Similarly, FIG. 11B illustrates the increased length of the first and second openings 164, 166, as compared to the shorter fixation element in FIG. 11A.

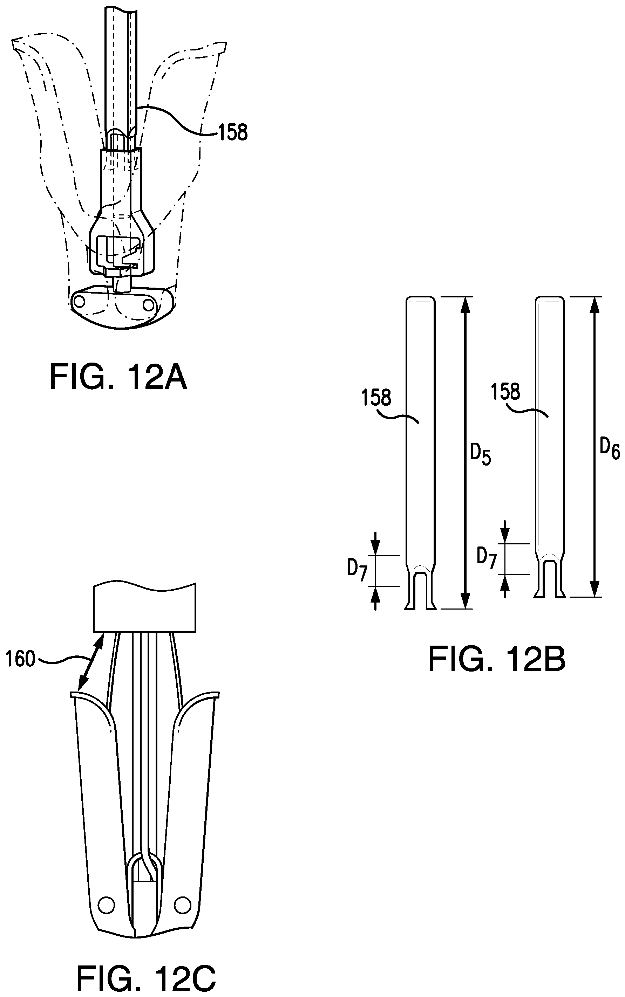

Referring now to FIGS. 12A-C, a portion of a system used to deliver the fixation device 102 to the desired position within body can include an L-Lock component 158, as shown in FIG. 12A. The L-Lock component can be elongated to accommodate the elongated fixation element 102 of the disclosed subject matter. For example, the length of the L-Lock can be increased from a dimension D₆ to a dimension D₅ to accommodate an elongated fixation device of the disclosed subject matter. For example, D₆ can be about 0.52 inches and D₅ can be about 14.2mm (0.56 inches). A U-shaped portion of the L-Lock can be the same dimension for both the shorter and elongated L-Lock, as shown by D₇. The elongated L-Lock thus can extend the fixation device 102 an increased distance from a distal end of the catheter to keep a gap 160 when the fixation elements are elongated. This can improve tissue clearance during the procedure.

To accommodate thicker leaflets when elongated fixation elements are present on the fixation device design, a longer L-Lock deployment mechanism may also be required to avoid any leaflet pinching against the radiopaque ring on the tip of the device delivery catheter. Leaflet thicknesses is typically below 2mm, but may exceed this in cases of myxotamous disease. Therefore, this potential "gap" accommodation can be required in cases where thicker leaflets are anticipated.

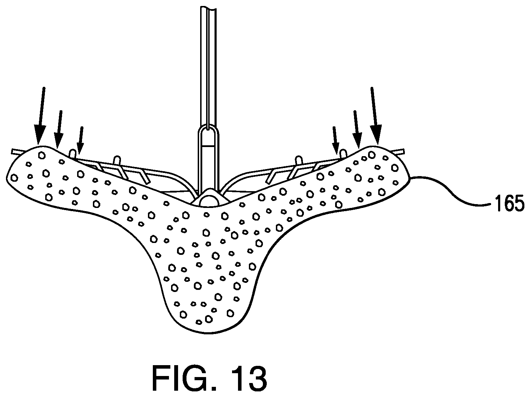

Referring now to FIG. 13, when gripping elements 110 and 112 are lowered toward respective fixation elements 104, 106 for tissue capture, the fixation elements and gripping elements can misfit by a 60-degree interference angle such that tissue can be pinched more immediately, completely, and forcefully at the free ends of the gripping elements. The arrows in FIG. 13 illustrate the larger amount of force that can be placed on tissue towards the free ends of the gripping elements. This engagement, which appears to be a mis-fitting and non-parallel assembly, provides a more secure fixation during fixation element closure and increases the likelihood that leaflets will not be released as the fixation device moves towards the closed position.

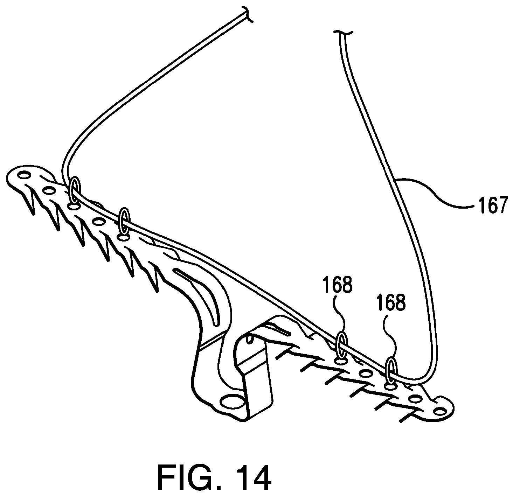

Referring now to FIG. 14, the gripping elements of the disclosed subject matter can each include more than one suture loop 168. As shown in FIG. 14 for purpose of illustration and not limitation, each gripping element can include two suture loops 168. The suture loops can be used in combination with sutures to raise and lower the gripping elements.

Additional details of suitable components and operation thereof along with related features are set forth in ITS, Pat. No. 7,226,467 to Lucatero et al., U.S. Pat, No. 7,563,267 to Goldfarb et ah, ll.S. Pat. No. 7,655,015 to Goldfarb et ah, U.S. Pat. No. 7,736,388 to Goldfarb et al., U.S. Pat. No. 7,811,296 to Goldfarb et al., U.S. Pat. No. 8,057,493 to Goldfarb et al., U.S. Pat. No. 8,303,608 to Goldfarb et al, U.S. Pat. No. 8,500,761 to Goldfarb et al., U.S. Pat. No. 8,734,505 to Goldfarb et al., U.S. Pat. No. 8,740,920 to Goldfarb et al., U.S. Pat. No. 9,510,829 to Goldfarb et al., U.S. Patent Publication No. 2007/0197858 to Goldfarb et. Al, U.S. Patent Application Publication No. 2017/0042546 to Goldfarb et al., and U.S. Patent Application Publication No. 2017/0239048 to Goldfarb et al.

The fixation device of the disclosed subject matter with gripper elements of greater length L₁ relative to the width W₁ and/or height H₁ surprisingly improves the function of a single fixation device and reduces the need to require more than one fixation device to be implanted to sufficiently reduce a patient's valvular regurgitation. For this reason, a fixation device that more completely reduces mitral regurgitation (MR) is desired, and a reduced number of fixation devices.

In patients with severe FMR, large annulus dilatation is chronically present, which causes otherwise healthy leaflets to fail to coapt or sea! during cardiac systole. As a result, mitral regurgitation occurs. In these cases, a large gap can occur between leaflets that can be difficult to bridge with a fixation device, even when opened to its maximum wingspan at 180 degrees opening angle. The fixation device disclosed herein addresses this need.

Likewise, in patients with severe DMR, chaotically flailing or prolapsing leaflets can be difficult to grasp or capture. In these cases, conventional fixation elements (e.g., clip arms) may not be long enough to fully stabilize the excessively mobile leaflets during grasping. The longer fixation elements of the disclosed subject matter adequately secures the leaflets for subsequent leaflet capture with the gripping element. In this way, the longer fixation elements disclosed herein can reduce the number of grasp and capture attempts required to achieve procedural success.

Also, when a fixation device is implanted in a regurgitant valve, a double orifice geometry is created. The fixation device disclosed herein therefore is configured to improve procedural success, without increasing the risk of obstruction due to the device. For the device disclosed herein, a computational model was used to evaluate the impact of the length of the fixation elements. In addition, the fixation element length was confirmed to be within the range of surgical stich lengths described in literature for treating severe leaflet prolapse (Barlow's Syndrome), or within 1 cm in length.

The elongated fixation device of the disclosed subject matter, further can improve tissue compression load at increased tissue thickness. For example, the gripping elements can be made of Nitinol which can have a stable pinching force over thin or thick leaflets versus a standard metal. Indeed, if a thick or thin leaflet is pinched in the gripper, it experiences a comparable force with nitinol. This is beneficial in a variety of situations, including in cases where a very thin delicate leaflet is inserted into the clip, but is folded over itself near the tip. This situation has been observed beating heart testing. When the leaflet is very thin, it is more prone to tearing under a pinch force, however, if it is doubled up, a typical elastic-plastic gripper deflects more and exerts more force on the vulnerable tissue, which can lead to piercing or tearing. The risk of tissue damage is mitigated through the use of a nitinol gripper where the pinching force remains stable despite any difference in tissue thickness. Therefore, the nitinol gripper design can minimize the occurrence of inadvertent device detachment from tissue (acute, subacute or even chronic) in what is defined as an SLDA (Single Leaflet Device Attachment - i.e., one side has detached) event.

The embodiments of the disclosed subject matter can be varied to treat any specific version of mitral valve, tricuspid valve, or other valves required edge-to edge approximation. For instance, various design features can be made "gentler" (reduced height of friction elements) for the purpose of treating a patient known to have fibroelastic deficiency or minor calcification. Alternatively, a more aggressive friction element height can be provided to more efficiently treat a myxomatous valve known to have thicker and fibrotic leaflet structures infiltrated with extracellular matrix.

Each of the components of the fixation device of the disclosed subject matter can be made of any suitable material (e.g., plastic, composites, metal, etc.) and technique for its intended purpose. In addition to the specific embodiments claimed below, the disclosed subject matter is also directed to other embodiments having any other possible combination of the dependent features claimed below and those disclosed above. As such, the particular features disclosed herein can be combined with each other in other manners within the scope of the claims such that the disclosed subject matter should be recognized as also specifically directed to other embodiments having any other possible combinations. Thus, the description of specific embodiments of the disclosed subject matter has been presented for purposes of illustration and description. It is not intended to be exhaustive or to limit the disclosed subject matter to those embodiments disclosed, with the invention being defined by the appended claims.

For purpose of understanding the assembly and operation of the disclosed subject matter, FIGS. 2-14 illustrate embodiments of components the fixation device 102 of FIG. 1 including in various possible positions during introduction and placement of the device 102 within the body to perform a therapeutic procedure. For example, FIG. 9A illustrates an embodiment of a device in a closed position for delivery through a catheter. It may be appreciated that the catheter may take the form of a guide catheter or sheath. As embodied herein, fixation elements can include engagement surfaces, wherein the engagement surfaces are configured to engage tissue between the gripping elements and the engagement surface. As shown in FIG. 9A, in the closed position, the opposed pair of fixation elements 134 are positioned so that engagement surfaces face each other. Each fixation element 104, 106 can have a cupped or concave shape so that together the arms surround a portion of the fixation device 102 and optionally contact each other on opposite sides of the device. This provides a low profile for the fixation device 102 which is readily passable through the catheter and through any anatomical structures, such as the mitral valve. In addition, the fixation device can further include an actuation mechanism. The actuation mechanism can comprise two legs 68 which are each movably coupled to a base 344. The base 144 is operatively connected with actuator rod which is used to manipulate the fixation device 102. At least a portion of the actuator rod can be disposed within the catheter and the distal end can attach to a stud which in turn is attached to the base 144. In some embodiments, the stud is threaded so that the actuator rod attaches to the stud by a screw-type action. However, the actuator rod and stud may be joined by any mechanism which is releasable to allow the fixation device 102 to be detached from the catheter.

FIG. 1 illustrates the fixation elements 104, 106 in the open position. In the open position, the fixation elements 104, 106 are rotated so that the engagement surfaces face a first direction. Distal advancement of the actuator rod causes a mechanism to engage the fixation elements 104,106 which begin to rotate around the first fixation element axis point 146 and the second fixation element axis point 148. Such rotation and movement of the fixation elements 146, 148 radially outward causes rotation of the legs 170, 172 about joints so that the legs are directly slightly outwards. The actuator rod can be advanced to any desired distance correlating to a desired separation of the fixation elements 104, 106. In the open position, engagement surfaces are disposed at an acute angle relative to a longitudinal center of the device, and are preferably at an angle of between 90 and 180 degrees relative to each other.

Gripping elements 110, 132 are typically biased outwardly towards the fixation elements 104, 106. The gripping elements 110, 112 can be moved inwardly toward the longitudinal center of the device and held with the aid of one or more sutures 167 which can be in the form of sutures, wires, nitinol wire, rods, cables, polymeric lines, or other suitable structures. The sutures 167 can be connected with the gripping elements by threading the sutures in a variety of ways. When the gripping elements 110, 112 have one or more suture loops, as shown in FIG. 14, the suture 167 can pass through the one or more loops on each gripping element and double back.

The embodiment illustrated herein are adapted for repair of the mitral valve using an antegrade approach from a patient's left atrium. The fixation device 102 is advanced through the mitral valve from the left atrium to the left ventricle. The distal fixation elements 104, 106 are oriented to be perpendicular to a line of coaptation and then positioned so that the fixation elements contact the ventricular surface of the valve leaflets, thereby grasping the leaflets. The gripping elements 110, 112 remain on the atrial side of the valve leaflets so that the leaflets lie between the gripping elements and the fixation elements. As embodied herein the gripping elements 110, 112 have friction elements 126, as shown in FIG. 2, such as barbs which are directed toward the fixation elements 104, 106. The friction elements 126 can be angled toward the respective gripping element first ends 114, 116. However, neither the gripping elements 110, 112 nor the friction elements 126 contact the leaflets at this time.

The fixation device 102 can be repeatedly manipulated to reposition the device so that the leaflets are properly contacted or grasped at a desired location. Repositioning is achieved with the fixation device in the open position. In some instances, regurgitation of the valve can also be checked while the device is in the open position. If regurgitation is not satisfactorily reduced, the device can be repositioned and regurgitation checked again until the desired results are achieved.

It can also be desired to invert the fixation device 102 to aid in the release of leaflets, repositioning, or removal of the fixation device 102. FIG. 8 illustrates the fixation elements in the inverted position. By further advancement of the actuator rod, the fixation elements can be further rotated so that engagement surfaces of the fixation elements 104, 106 face outwardly and second ends 134, 336 point distally, with each fixation element 110,112 forming an obtuse angle relative to the central axis. The angle between fixation elements 110, 112 is preferably in the range of about 270 to 360 degrees. Further advancement of the actuator rod further rotates the fixation elements 104, 106 around axis point 146, 148. This rotation and movement of the fixation elements 104, 106 radially outward causes rotation of the legs about joints so that the legs are returned toward an initial position, generally parallel to each other. The actuator rod can be advanced to any desired distance correlating to a desired inversion of the fixation elements 104, 106. Thus, a relatively large space can be created between the elements for repositioning. In addition, the inverted position allows withdrawal of the fixation device across the valve while minimizing trauma to the leaflets. Engagement surfaces provide an atraumatic surface for deflecting tissue as the fixation device is retracted proximally. The engagement surface can be on a portion of the fixation device capable of independent movement from the friction elements. As such. It should be further noted that friction elements are angled slightly to reduce the risk that the friction elements will catch on or lacerate tissue as the fixation device is withdrawn.

Once the fixation device 14 has been positioned in a desired location relative to the valve leaflets, the leaflets can then be captured between the gripping elements 110, 112 and the fixation elements 104, 106. At this time, the gripping elements 110,112 are lowered toward the fixation elements 134, 136 so that the leaflets are held therebetween. At any time, the gripping elements 110, 112 can be raised and the fixation elements 104, 106 adjusted or inverted to reposition the fixation device 102, if regurgitation is not sufficiently reduced.

After the leaflets have been captured between the gripping elements and fixation elements in a desired arrangement, the fixation elements can be locked to hold the leaflets in this position or the fixation device can be returned to or toward a closed position, FIG. 9A illustrates the fixation elements in the closed position wherein the leaflets (not shown) can be captured and coapted. This is achieved by retraction of the actuator rod proximally relative to so that the legs of the actuation mechanism apply an upwards force to the fixation elements, which in turn rotate the fixation elements so that the engagement surfaces again face one another. The released gripping elements 110, 112 which are biased outwardly toward fixation elements 104, 106 are concurrently urged inwardly by the fixation elements 104, 106. The fixation device can then be locked to hold the leaflets in this dosed position.

The fixation device 102 can then be released from the catheter. As mentioned, the fixation device 102 is releasably coupleable to the catheter. FIGS. 12A-12B illustrate a portion of the coupling mechanism, L-Lock 158. The L-Lock can be used in conjunction with a threaded mechanism to uncouple the fixation device 102 from the catheter.

After detachment, the repair of the leaflets or tissue can be observed by non-invasive visualization techniques, such as echocardiography, to ensure the desired outcome. If the repair is not desired, the fixation device 14 can be retrieved such as by the use of the sutures 167 so as to reconnect the fixation device 102 with the catheter. If the repair is desired, the sutures 167 can be disconnected by releasing one end of each line at the proximal end of the catheter and the other end pulled to draw the free end of the suture distally through the catheter and through the engagement with the gripping elements 110, 112.

While the above described embodiments of the invention utilize a push-to-open, pull-to-close mechanism for opening and closing fixation elements 104, 106, it should be understood that a pull-to-open, push-to-close mechanism is equally possible.

While the disclosed subject matter is described herein in terms of certain preferred embodiments for purpose of illustration and not limitation, those skilled in the art will recognize that various modifications and improvements can be made to the disclosed subject matter without departing from the scope of the claims. Moreover, although individual features of one embodiment of the disclosed subject matter can be discussed herein or shown in the drawings of one embodiment and not in other embodiments, it should be readily apparent that individual features of one embodiment can be combined with one or more features of another embodiment or features from a plurality of embodiments, within the scope of the appended claims.

In addition to the specific embodiments claimed below, the disclosed subject matter is also directed to other embodiments having any other possible combination of the dependent features claimed below and those disclosed above. As such, the particular features presented in the dependent claims and disclosed above can be combined with each other in other possible combinations, within the scope of the appended claims. Thus, the foregoing description of specific embodiments of the disclosed subject matter has been presented for purposes of illustration and description. It is not intended to be exhaustive or to limit the disclosed subject matter to those embodiments disclosed, with the invention being defined by the appended claims.

## Claims

1. A fixation device (102) for engaging tissue comprising:
a pair of fixation elements (104,106), each fixation element (104,106) having a first end (130,132) and a second end (134,136) opposite the first end (130,132), the first ends (130,132) being moveable between a closed position and an open position,
a pair of gripping element flat sections (110,112), each gripping element flat section (110,112) moveable with respect to a respective fixation element (104,106) and disposed in opposition to at least a portion of the respective fixation element (104,106) to capture tissue therebetween, with the first ends (130,132) of the fixation elements (104, 106) being in the open position; and
a central portion (108) operatively connected to each gripping element flat section (110,112) at a respective central portion-gripping element interface (122,124), the central portion (108) having a distal end (128), the central portion (108) having a width "W₁" proximate the distal end (128);
each gripping element flat section (110,112) having a free end (118,120) opposite its respective central portion-gripping element interface (122,124) and a length "L₁" defined between the respective central portion-gripping element interface (1422,124) and the free end (118,120);
wherein the distal end (128) defines a reference plane (140) perpendicular to a central axis of the central portion and wherein a height "H₁" is defined by a vertical dimension between the reference plane (140) and the central portion-gripping element interfaces (122,124);
**characterized in that** the ratio of the width to the length W₁:L₁ is about 1:3 and the ratio of the height to the length H₁:L₁ is about 1:1.8.

2. The fixation device (102) of claim 1, wherein the length L₁ is about 8.9mm (0.35 inches).

3. The fixation device (102) of claim 1 or 2, the central portion (108) having a generally U-shaped configuration with the distal end (128) disposed between the respective central portion-gripping element interfaces (122,124).

4. The fixation device (102) of any of claims 1-63, wherein each fixation element (104,106) is rotatable about a respective axis point (146,148), each fixation element (104,106) having an elongate portion (150,152) defining a respective reference axis, wherein a length "L₂" is defined along the respective reference axis between the respective axis point (146,148) and the respective second end (134,136) of each fixation element (104,106).

5. The fixation device (102) of claim 4, wherein the length L₂ is at least the length of L₁.

6. The fixation device (102) of claim 4 or 5, wherein the ratio of the lengths L₂:L₁ is about 1.35:1.

7. The fixation device (102) of any of claims 1-96, each gripping element flat section (110,112) has at least four rows of friction elements (126) extending from a gripping element surface.

8. The fixation device (102) of claim 7, wherein each row includes at least one friction element (126).

## Patentansprüche

1. Befestigungsvorrichtung (102) zum Eingriff in Gewebe, die umfasst:
ein Paar Befestigungselemente (104, 106), wobei jedes Befestigungselement (104, 106) ein erstes Ende (130, 132) und ein dem ersten Ende (130, 132) gegenüberliegendes zweites Ende (134, 136) aufweist, wobei die ersten Enden (130, 132) zwischen einer geschlossenen Position und einer offenen Position bewegbar sind;
ein Paar flacher Abschnitte (110, 112) eines Greifelements, wobei jeder flache Abschnitt (110, 112) eines Greifelements relativ zu einem jeweiligen Befestigungselement (104, 106) beweglich ist und gegenüber mindestens einem Abschnitt des jeweiligen Befestigungselements (104, 106) angeordnet ist, um Gewebe dazwischen einzuklemmen, wobei sich die ersten Enden (130, 132) der Befestigungselemente (104, 106) in der offenen Position befinden; und
einen Mittelabschnitt (108), der an einer jeweiligen Schnittstelle (122, 124) zwischen Mittelabschnitt und Greifelement funktionsmäßig mit jedem flachen Abschnitt (110, 112) eines Greifelements verbunden ist, wobei der Mittelabschnitt (108) ein distales Ende (128) aufweist, wobei der Mittelabschnitt (108) nahe dem distalen Ende (128) eine Breite "W₁" aufweist;
wobei jeder flache Abschnitt (110, 112) eines Greifelements ein freies Ende (118, 120) aufweist, das der jeweiligen Schnittstelle (122, 124) zwischen Mittelabschnitt und Greifelement gegenüberliegt, und eine Länge "L1", die zwischen der jeweiligen Schnittstelle (122, 124) zwischen Mittelabschnitt und Greifelement und dem freien Ende (118, 120) definiert ist;
wobei das distale Ende (128) eine Bezugsebene (140) senkrecht zu einer Mittelachse des Mittelabschnitts definiert und wobei eine Höhe "H₁" durch eine vertikale Abmessung zwischen der Bezugsebene (140) und den Schnittstellen (122, 124) zwischen Mittelabschnitt und Greifelement definiert ist;
**dadurch gekennzeichnet, dass** das Verhältnis von Breite zu Länge W₁:L₁ etwa 1:3 und das Verhältnis von Höhe zu Länge H₁:L₁ etwa 1:1,8 beträgt.

2. Befestigungsvorrichtung (102) nach Anspruch 1, wobei die Länge L₁ etwa 8,9 mm (0,35 Zoll) beträgt.

3. Befestigungsvorrichtung (102) nach Anspruch 1 oder 2, wobei der Mittelabschnitt (108) eine im Wesentlichen U-förmige Konfiguration aufweist wobei das distale Ende (128) zwischen den jeweiligen Schnittstellen (122, 124) zwischen Mittelabschnitt und Greifelement angeordnet ist.

4. Befestigungsvorrichtung (102) nach einem der Ansprüche 1-3, wobei jedes Befestigungselement (104, 106) um einen jeweiligen Achspunkt (146, 148) drehbar ist, wobei jedes Befestigungselement (104, 106) einen länglichen Abschnitt (150, 152) aufweist, der eine jeweilige Referenzachse definiert, wobei eine Länge "L₂" entlang der jeweiligen Referenzachse zwischen dem jeweiligen Achspunkt (146, 148) und dem jeweiligen zweiten Ende (134, 136) jedes Befestigungselements (104, 106) definiert ist.

5. Befestigungsvorrichtung (102) nach Anspruch 4, wobei die Länge L₂ mindestens der Länge von L₁ entspricht.

6. Befestigungsvorrichtung (102) nach Anspruch 4 oder 5, wobei das Verhältnis der Längen L₂:L₁ etwa 1,35:1 beträgt.

7. Befestigungsvorrichtung (102) nach einem der Ansprüche 1-6, wobei jeder flache Abschnitt (110, 112) eines Greifelements mindestens vier Reihen von Reibungselementen (126) aufweist, die sich von einer Oberfläche des Greifelements erstrecken.

8. Befestigungsvorrichtung (102) nach Anspruch 7, wobei jede Reihe mindestens ein Reibungselement (126) enthält.

## Revendications

1. Dispositif de fixation (102) pour entrer en prise avec un tissu, comprenant :
une paire d'éléments de fixation (104, 106), chaque élément de fixation (104, 106) ayant une première extrémité (130, 132) et une seconde extrémité (134, 136) opposée à la première extrémité (130, 132), les premières extrémités (130, 132) étant mobiles entre une position fermée et une position ouverte ;
une paire de sections plates d'élément d'adhérence (110, 112), chaque section plate d'élément d'adhérence (110, 112) étant mobile par rapport à un élément de fixation respectif (104, 106) et disposée en opposition à au moins une partie de l'élément de fixation respectif (104, 106) pour capturer un tissu entre ceux-ci, les premières extrémités (130, 132) des éléments de fixation (l 04, 106) étant dans la position ouverte ; et
une partie centrale (108) raccordée opérationnellement à chaque section plate d'élément d'adhérence (110, 112) au niveau d'une interface respective partie centrale-élément d'adhérence (122, 124), la partie centrale (108) ayant une extrémité distale (128), la partie centrale (108) ayant une largeur « W₁ » à proximité de l'extrémité distale (128) ;
chaque section plate d'élément d'adhérence (110, 112) ayant une extrémité libre (118, 120) opposée à son interface respective partie centrale-élément d'adhérence (122, 124) et une longueur « L₁ » définie entre l'interface respective partie centrale-élément d'adhérence (122, 124) et l'extrémité libre (118, 120) ;
dans lequel l'extrémité distale (128) définit un plan de référence (140) perpendiculaire à un axe central de la partie centrale et dans lequel une hauteur « H₁ » est définie par une dimension verticale entre le plan de référence (140) et l'interface partie centrale-élément d'adhérences (122, 124) ;
**caractérisé en ce que** le rapport de la largeur à la longueur W₁ : L₁ est environ 1 : 3 et le rapport de la hauteur à la longueur H₁ : L₁ est environ 1 : 1,8.

2. Dispositif de fixation (102) de la revendication 1, dans lequel la longueur L₁ est d'environ 8,9 mm (0,35 pouces).

3. Dispositif de fixation (102) de la revendication 1 ou 2, la partie centrale (108) ayant une configuration généralement en forme de U avec l'extrémité distale (128) disposé entre les interfaces respectives partie centrale-élément d'adhérence (122, 124).

4. Dispositif de fixation (102) de quelconques des revendications précédentes 1 à 3, dans lequel chaque élément de fixation (104, 106) est rotatif autour d'un point d'axe respectif (146, 148), chaque élément de fixation (104, 106) ayant une partie oblongue (150, 152) définissant un axe de référence respectif, dans lequel une longueur « L₂ » est définie le long de l'axe de référence respectif entre le point d'axe respectif (146, 148) et la seconde extrémité respective (134, 136) de chaque élément de fixation (104, 106).

5. Dispositif de fixation (102) de la revendication 4, dans lequel la longueur L₂ est au moins la longueur de L₁.

6. Dispositif de fixation (102) de la revendication 4 ou 5, dans lequel le rapport des longueurs L₂ : L₁ est environ 1,35 : 1.

7. Dispositif de fixation (102) de quelconques des revendications précédentes 1 à 6, chaque section plate d'élément d'adhérence (110, 112) a au moins quatre rangées d'éléments de friction (126) s'étendant depuis une surface d'élément d'adhérence.

8. Dispositif de fixation (102) de la revendication 7, dans lequel chaque rangée inclut au moins un élément de friction (126).
